# EUROPEAN PATENT APPLICATION

(11) **EP 1 433 789 A1**
(43) Date of publication of application: **30.06.2004**
(21) Application number: 02406143.4
(22) Date of filing: 24.12.2002
(51) Int. Cl.: C07D 471/14, C07D 491/22, A61K 31/4985, A61P 35/00

(54) **Pyrrolopyrazines and their use as selective apoptosis inducers**

(71) Applicant: Aponetics AG, 4108 Witterswil (CH)
(72) Inventor: Bachmann, Felix, 4052 Basel (CH); Eberle, Martin, 4103 Bottmingen (CH); Strebel, Alessandro, 4104 Oberwil (CH)
(74) Representative: Becker, Konrad, Dr.

(57) **Abstract**

Substituted pyrrolopyrazines of formula I 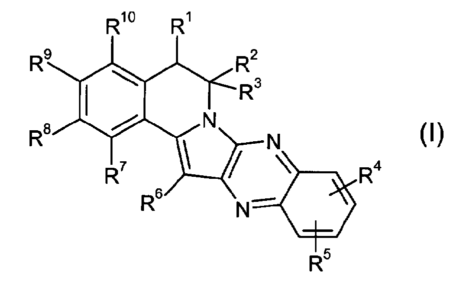 are selectively inducing apoptosis in cancer cells, and can be used for the treatment of neoplastic and autoimmune diseases. The invention relates to novel compounds of formula I wherein the substituents R¹ to R¹⁰ are as defined in the specification, to methods of synthesis of such compounds, to compounds of formula I for use as medicaments, to pharmaceutical compositions containing compounds of formula I, to the use of a compounds of formula I for the preparation of a pharmaceutical composition for the treatment of neoplastic and autoimmune diseases, and to methods of treatment of neoplastic and autoimmune diseases using such compounds of formula I or of pharmaceutical compositions containing same.

## Description

The invention relates to novel substituted pyrrolopyrazines, processes for the preparation thereof, pharmaceutical compositions containing same, the use thereof optionally in combination with one or more other pharmaceutically active compounds for the therapy of neoplastic diseases, disorders of the immune system, degenerative diseases and transplantation related pathologies, and a method for the treatment of such a disease.

### Background of the invention

Cancer is one of the leading causes of death in humans. Although a variety of drugs against neoplastic diseases have been developed and techniques are available such as surgery and radiation therapy, there is still a need for alternative and improved methods of treatment of neoplastic diseases.

Autoimmune diseases are associated with abnormal lymphoproliferation as a result of defects in the termination of lymphocyte activiation and growth. Often, such diseases are associated with inflammation like rheumatoid arthritis, insulin dependent diabetes mellitus, multiple sclerosis, systemic lupus erythematosus and the like. The treatment of such diseases is focused on anti-inflammatory and immunosuppressive drugs which in a lot of cases show severe side effects. Hence, there is a need for alternative drugs with a new mode of action showing less side effects.

Apoptosis is a term used to describe a series of cellular events which occur to bring about programmed cell death. There are various apoptotic pathways, some of which have been characterized, whereas others remain to be elucidated. If the balance between cell division and apoptosis is disturbed, life-threatening diseases including cancer, autoimmune disorders, neurodegenerative and cardiovascular diseases may occur.

In recent years it has become clear that programmed cell death (apoptosis) is as important to the health of a multicellular organisms as cell division. By repeated cell division and differentiation throughout development or tissue repair, surplus or even harmful cells are generated. In order to maintain tissue homeostasis these cells have to be removed or killed. The delicate interplay between cell growth and apoptosis in an organism is mirrored in the complex molecular balance that determines whether an individual cell undergoes division, arrests in the cell cycle or commits to programmed cell death.

Dysregulation of cell proliferation, or lack of appropriate cell death, has wide ranging clinical implications. A number of diseases associated with such dysregulation involve hyperproliferation, inflammation, tissue remodeling and repair. Familiar indications in this category include cancers, restenosis, neointimal hyperplasia, angiogenesis, endometriosis, lymphoproliferative disorders, transplantation related pathologies (graft rejection), polyposis, loss of neural function in the case of tissue remodeling and the like. Such cells may lose the normal regulatory control of cell division, and may also fail to undergo appropriate cell death.

As apoptosis is inhibited or delayed in most types of proliferative, neoplastic diseases, induction of apoptosis is an option for treatment of cancer, especially in cancer types which show resistance to classic chemotherapy, radiation and immunotherapy (Apoptosis and Cancer Chemotherapy, Hickman and Dive, eds., Blackwell Publishing, 1999). Also in autoimmune and transplantation related diseases and pathologies compounds inducing apoptosis may be used to restore normal cell death processes and therefore can eradicate the symptoms and might cure the diseases. Further applications of compounds inducing apoptosis may be in restenosis, i.e. accumulation of vascular smooth muscle cells in the walls of arteries, and in persistent infections caused by a failure to eradicate bacteria- and virus-infected cells. Furthermore, apoptosis can be induced or re-established in epithelial cells, in endothelial cells, in muscle cells, and in others which have lost contact with extracellular matrix. These cells are potentially able to colonize other organs and therefore can develop into pathologies like neoplasias, endometriosis and the like.

In US Patent 5,910,590 and related patents Blum and Hutchinson have described various pyrrolopyrazines and their use as selective agonists or antagonists for GABA brain receptors for the diagnosis and treatment of anxiety, sleep and seizure disorders.

In a study of the reaction of enamines of isoquinoline type with oxalyl chloride Mikhailovskii and Shklyaev (Chemistry of Heterocyclic Compounds 30, 818 (1994)) obtained dioxopyrroloisoquinolines which were characterized by reaction with o-phenylenediamine to give penta- and hexacyclic pyrrolopyrazines. However, these authors did not recognize the potential of such pyrrolopyrazines as pharmaceuticals for the treatment of neoplastic and autoimmune diseases.

### Summary of the invention

Substituted pyrrolopyrazines of formula I are selectively inducing apoptosis in cancer cells, and can be used for the treatment of neoplastic and autoimmune diseases. The invention relates to novel compounds of formula I as defined hereinafter, to methods of synthesis of such compounds, to compounds of formula I for use as medicaments, to pharmaceutical compositions containing compounds of formula I, to the use of a compounds of formula I for the preparation of a pharmaceutical composition for the treatment of neoplastic and autoimmune diseases, and to methods of treatment of neoplastic and autoimmune diseases using such compounds of formula I or of pharmaceutical compositions containing same.

### Detailed description of the invention

The invention relates to compounds of formula 1, 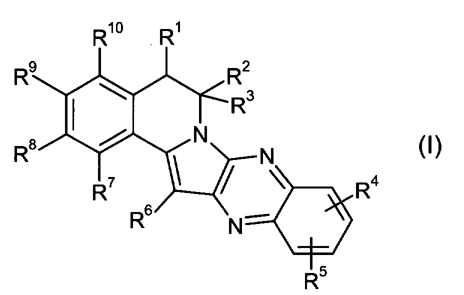 wherein
R¹ represents hydrogen, lower alkyl, lower alkoxy-lower alkyl, halo-lower alkyl or optionally substituted phenyl;
R² represents hydrogen, lower alkyl, lower alkoxy-lower alkyl, halo-lower alkyl, optionally substituted phenyl, or R¹ and R² together with the bond connecting them represent a carbocyclic ring;
R³ represents hydrogen or lower alkyl, or R² and R³ together with the bond connecting them represent a carbocyclic ring;
R⁴ represents hydrogen, lower alkyl, halo-lower alkyl, lower alkoxy, lower alkoxy-lower alkoxy or optionally substituted phenyl;
R⁵ represents hydrogen, lower alkyl, halo-lower alkyl, lower alkoxy, or R⁴ and R⁵ in ortho position to each other represent methylenedioxy or together with the bond connecting them represent an annulated aromatic or aliphatic ring;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl;
R⁷, R⁸, R⁹ and R¹⁰, independently of each other, represent hydrogen, lower alkyl, halogen, haloalkyl, lower alkoxy, lower alkylthio, lower alkoxy-lower alkoxy, aminoalkoxy wherein the nitrogen atom is unsubstituted or substituted by lower alkyl, hydroxy-lower alkyl or lower alkoxy-lower alkyl or wherein the nitrogen atom is part of heterocyclyl, lower acyloxy, lower alkoxy-lower acyloxy, or di-lower alkylamino-lower acyloxy; or also R⁷ and R⁸, or R⁸ and R⁹, or R⁹ and R¹⁰ together represent methylenedioxy or an annulated aromatic or aliphatic ring; with the proviso that at least one of the substituents R⁷, R⁸, R⁹ and R¹⁰ represents hydrogen;
and salts thereof.

Excluded are the compounds of formula I wherein R¹ is hydrogen, R² and R³ are both methyl, R⁴, R⁵, R⁶ and R⁷ are hydrogen, R⁸ and R⁹ are both hydrogen or both methoxy, and R¹⁰ is hydrogen; the compound of formula I wherein R¹ is hydrogen, R² and R³ together represent tetramethylene, and R⁴, R⁵, R⁶, R⁷, R⁸, R⁹, and R¹⁰ are hydrogen; and the compound of formula I wherein R¹ and R² together represent tetramethylene, R³ is methyl, and R⁴, R⁵, R⁶, R⁷, R⁸, R⁹, and R¹⁰ are hydrogen;

The general terms used hereinbefore and hereinafter preferably have within the context of this disclosure the following meanings, unless otherwise indicated:

The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Any asymmetric carbon atoms may be present in the (R)-, (S)- or (R,S)-configuration, preferably in the (R)- or (S)-configuration. The compounds may thus be present as mixtures of isomers or as pure isomers, preferably as enantiomer-pure diastereomers.

The invention relates also to possible tautomers of the compounds of formula I.

Alkyl is preferably alkyl with from and including 1 up to and including 12, preferably from 1 to 6, carbon atoms, and is linear or branched. Alkyl is preferably lower alkyl.

Lower alkyl is butyl, such as n-butyl, sec-butyl, isobutyl, tert-butyl, propyl, such as n-propyl or isopropyl, ethyl or methyl. Preferably lower alkyl is methyl or ethyl.

A carbocyclic ring is cycloalkyl or phenyl. Cycloalkyl has preferably 3 to 7 ring carbon atoms, and may be unsubstituted or substituted, e.g. by lower alkyl or lower alkoxy. Cycloalkyl is, for example, cyclohexyl, cyclopentyl, or methylcyclopentyl.

An aliphatic ring is preferably a 5-, 6- or 7-membered ring containing optionally one or two heteroatoms selected from nitrogen, oxygen and sulfur. Preferably an aliphatic ring is cyclopentane, cyclohexane, cycloheptane, dihydrofuran, tetrahydrofuran, tetrahydropyran, dioxolane, dioxane, pyrrolidine, piperidine, morpholine or piperazine, optionally substituted by one or more substituents selected from lower alkyl, lower alkoxy, oxo or aryl.

An aromatic ring is a 5- or 6-membered ring containing optionally one oxygen atom, one sulfur atom, and/or one, two or three nitrogen atoms. Preferably an aromatic ring is benzene, furan, thiophene, pyrrole, pyrazole, imidazole, triazole, oxazole, thiazole, pyridine, pyridazine, pyrimidine, or pyrazine, optionally substituted by one or more substituents selected from lower alkyl, lower alkoxy or aryl.

Aryl is preferably a 5- or 6-membered aromatic ring as defined hereinbefore, or a bi- or tricyclic aromatic residue containing 9 to 14 ring members and aromatic or aliphatic rings as defined hereinbefore wherein at least one of the rings is an aromatic ring. Examples for a bi- or tricyclic aromatic residue as aryl are naphthalene, tetrahydronaphthalene, phenanthrene, indole, benzimidazole, benzpyrane, quinoline, isoquinoline, or purine.

Arylalkyl is preferably optionally substituted phenyl-lower alkyl, in particular benzyl or 2-phenylethyl.

In optionally substituted alkenyl or alkinyl, substituents are preferably lower alkyl, lower alkoxy, hydroxy-lower alkyl, lower alkoxy-lower alkyl, optionally substituted amino-lower alkyl or di-lower alkylamino, and are connected with a saturated or an unsaturated carbon atom of alkenyl or alkinyl.

In optionally substituted phenyl, substituents are preferably lower alkyl, lower alkoxy, lower alkoxy-lower alkoxy, methylenedioxy, halo-lower alkyl, lower alkoxy-lower alkyl, halo, or nitro.

Nitrogen-containing heterocyclyl is preferably an aliphatic ring as defined hereinbefore containing at least one nitrogen atom, and is for example pyrrolidine, oxazolidine, thiazolidine, piperidine, morpholine, or piperazine. Such heterocyclyl may be attached through a carbon or a nitrogen atom.

Lower acyl is preferably lower alkylcarbonyl, in particular acetyl.

Hydroxyalkyl is especially hydroxy-lower alkyl, preferably hydroxymethyl, 2-hydroxyethyl or 2-hydroxy-2-propyl.

Haloalkyl is preferably fluoroalkyl, especially trifluoromethyl or pentafluoroethyl.

Halogen is fluorine, chlorine, bromine, or iodine.

Lower alkoxy is especially methoxy, ethoxy, isopropyloxy, or tert-butyloxy.

Lower alkylthio is especially methylthio or ethylthio.

Salts are especially the pharmaceutically acceptable salts of compounds of formula I.

Such salts are formed, for example, as acid addition salts, preferably with organic or inorganic acids. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

The compound of the formula I may be administered in the form of a pro-drug which is broken down in the human or animal body to give a compound of the formula I. Examples of pro-drugs include in vivo hydrolysable esters of a compound of the formula I.

The compounds of formula I have valuable pharmacological properties. The invention also relates to compounds of formula I as defined hereinbefore, including the compounds disclaimed as such, for use as medicaments.

The efficacy of the compounds of the invention in inducing apoptosis in tumor cells can be demonstrated as follows:

Relative fluorescent activities of suitable tumor cell lines transfected with green fluorescent protein (GFP) are measured in the presence of compounds of the invention and of standard tumor drugs, using the method described in WO 99/35493. Suitable tumor cell lines are A20.2J, a BALB/c B cell lymphoma, PB-3c, an IL-3 dependent, non tumorigenic mastocyte line isolated from the bone marrow of a DBA/2 mouse, Jurkat, a human acute T cell leukemia cell line, K562, a human chronic myelogenous leukemia cell line, HL60, a human acute promyelocytic leukemia cell line, Ramos and Raji, human B-cell lymphoma cell lines, H9 and Hut78, human T-cell lymphoma cell lines, HeLa and KB, human squamous cell carcinoma cell lines, MCF7, SK-BR-3, PC3, HBL-100, SW480, H460 and H1792, human adenocarcinoma cell lines and HT-1080, a human fibrosarcoma cell line.

Preferred standard drugs as compounds for comparisons are: a) antimetabolites such as 5-fluorouracil (ICN), gemcitabine HCI (Gemzar™, Eli Lilly), b) alkylating agents such as oxaliplatin (Eloxantin™, Sanofi-Synthelabo), dacarbazin (Detimedac™, Medac), cyclophosphamide (Endoxan™, Asta) and carboplatin (Paraplatin™, Bristol-Meyers Squibb), c) cell-cycle inhibitor such as vinorelbine (Navelbine™, Robapharm), vinblastine (Velbe™, Eli Lily), docetaxel (Taxotere™, Aventis), d) DNA breaker (topo-isomerase inhibitor, intercalator, strand breaker) such as doxorubicin HCI (Adriblastin™, Pharmacia-Upjohn), bleomycin (Asta-Medica), irinotecan (Campto™, Aventis), etoposide phosphate (Etopophos™, Bristol-Meyers Squibb), topotecan HCl, (Hycamtin™, GlaxoSmithKline), e) mixtures thereof, f) compounds interfering with the signal transduction pathway, such as caspase activity modifiers, agonists and antagonists of cell death receptors, modifiers of nucleases, phosphatases and kinases such as imatinib mesylate (Gleevec™, Novartis), dexamethasone, phorbol myristate acetate, cyclosporin A, quercetin, tamoxifen (Alexis Corporation, Switzerland).

Apoptosis is determined in a primary screen using a fluorescence plate reader and then in a secondary screen using FACS (fluorescence activated cell scanning). Compounds causing apoptosis without substantial cytotoxic side effects are chosen for further testing and characterization by using a combination of the following well established assays: A) Nuclear staining with Hoechst 33342 dye providing information about nuclear morphology and DNA fragmentation which are hallmarks of apoptosis. B) MTS proliferation assay measuring the metabolic activity of cells. Viable cells are metabolically active whereas cells with compromised respiratory chain show a reduced activity in this test. C) AnnexinV binding assay which reflects the phosphatidylserine content of the outer lipid bilayer of the plasma membrane. This event is considered an early hallmark of apoptosis. D) PI staining for cell cycle distribution which shows any alterations in the distribution among the different phases of the cell cycle. Cell cycle arresting points can be determined. E) Proliferation assay monitoring DNA synthesis by incorporating bromodeoxyuridine (BrdU). Inhibitory effects on growth/proliferation can be directly determined. F) Cystein proteinase dependency, respectively caspase dependency are determined by using specific inhibitors. This provides information about possible involvement of specific proteases in the mechanisms.

On the basis of these studies, a compound of formula I according to the invention shows therapeutic efficacy especially against neoplastic diseases and autoimmune diseases. In particular, the compounds of the invention are active against malignancies, e.g. epithelial neoplasms, squamous cell neoplasms, basal cell neoplasms, transitional cell papillomas and carcinomas, adenomas und adenocarcinomas, adnexal and skin appendage neoplasms, mucoepidermoid neoplasms, cystic neoplasms, mucinous and serous neoplasms, ductal-, lobular and medullary neoplasms, acinar cell neoplasms, complex epithelial neoplasms, specialized gonadal neoplasms, paragangliomas and glomus tumors, naevi and melanomas, soft tissue tumors and sarcomas, fibromatous neoplasms, myxomatous neoplasms, lipomatous neoplasms, myomatous neoplasms, complex mixed and stromal neoplasms, fibroepithelial neoplasms, synovial like neoplasms, mesothelial neoplasms, germ cell neoplasms, trophoblastic neoplasms, mesonephromas, blood vessel tumors, lymphatic vessel tumors, osseous and chondromatous neoplasms, giant cell tumors, miscellaneous bone tumors, odontogenic tumors, gliomas, neuroepitheliomatous neoplasms, meningiomas, nerve sheath tumors, granular cell tumors and alveolar soft part sarcomas, Hodgkin's and non Hodgkin's lymphomas, other lymphoreticular neoplasms, plasma cell tumors, mast cell tumors, immunoproliferative diseases, leukemias, miscellaneous myeloproliferative disorders, lymphoproliferative disorders and myelodysplastic syndromes.

The compounds of the invention are likewise active against autoimmune diseases, e.g. against systemic, discoid or subacute cutaneous lupus erythematosus, rheumatoid arthritis, anti phospholipid syndrome, CREST, progressive systemic sclerosis, mixed connective tissue disease (Sharp syndrome), Reiter's syndrome, juvenile arthritis, cold agglutinin disease, essential mixed cryoglobulinemia, rheumatic fever, ankylosing spondylitis, chronic polyarthritis, myasthenia gravis, multiple sclerosis, chronic inflammatory demyelinating polyneuropathy, Guillan-Barré syndrome, dermatomyositis/polymyositis, autoimmune hemolytic anemia, thrompocytopenic purpura, neutropenia, type I diabetes mellitus, thyroiditis (including Hashimoto's and Grave' disease), Addison's disease, polyglandular syndrome, pemphigus (vulgaris, foliaceus, sebaceous and vegetans), bullous and cicatricial pemphigoid, pemphigoid gestationis, epidermolysis bullosa acquisita, linear IgA disease, lichen sclerosus et atrophicus, morbus Duhring, psoriasis vulgaris, guttate, generalized pustular and localized pustular psoriasis, vitiligo, alopecia areata, primary biliary cirrhosis, autoimmune hepatitis, all forms of glomerulonephritis, pulmonal hemorrhage (goodpasture syndrome), IgA nephropathy, pernicious anemia and autoimmune gastritis, inflammatory bowel diseases (including colitis ulcerosa and morbus Crohn), Behcet's disease, Celic-Sprue disease, autoimmune uveitis, autoimmune myocarditis, granulomatous orchitis, aspermatogenesis without orchitis, idiopatic and secondary pulmonary fibrosis, inflammatory diesases with a possibility of autoimmune pathogensesis, such as pyoderma gangrensosum, lichen ruber, sarcoidosis (including Löfgren and cutaneous/subcutaneous type), granuloma anulare, allergic type I and type IV immunolgical reaction, asthma bronchiale, pollinosis, atopic, contact and airborne dermatitis, large vessel vasculitis (giant cell and Takayasu's arteritis), medium sized vessel vasculitis (polyarteritis nodosa, Kawasaki disease), small vessel vasculitis (Wegener's granulomatosis, Churg Strauss syndrome, microscopic polangiitis, Henoch-Schoenlein purpura, essential cryoglobulinemic vasculitis, cutaneous leukoklastic angiitis), hypersensitivity syndromes, toxic epidermal necrolysis (Stevens-Johnson syndrome, erythema multiforme), diseases due to drug side effects, all forms of cutaneous, organ-specific and systemic effects due to type I-VI (Coombs classification) immunologic forms of reaction, transplantation related pathologies, such as acute and chronic graft versus host and host versus graft disease, involving all organs (skin, heart, kidney, bone marrow, eye, liver, spleen, lung, muscle, central and peripheral nerve system, connective tissue, bone, blood and lymphatic vessel, genito-urinary system, ear, cartillage, primary and secondary lymphatic system including bone marrow, lymph node, thymus, gastro-intestinal tract, including oro-pharynx, esophageus, stomach, small intestine, colon, and rectum, including parts of above mentioned organs down to single cell level and substructures, e.g. stem cells).

A compound of formula I can be administered alone or in combination with one or more other therapeutic agents, possible combination therapy taking the form of fixed combinations, or the administration of a compound of the invention and one or more other therapeutic agents being staggered or given independently of one another, or the combined administration of fixed combinations and one or more other therapeutic agents. A compound of formula I can, besides or in addition, be administered especially for tumor therapy in combination with chemotherapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk. Particularly preferred is the use of compounds of formula I in combination with radiotherapy.

Therapeutic agents for possible combination are especially one or more cytostatic or cytotoxic compounds, for example a chemotherapeutic agent or several selected from the group comprising indarubicin, cytarabine, interferon, hydroxyurea, bisulfan, or an inhibitor of polyamine biosynthesis, an inhibitor of protein kinase, especially of serine/threonine protein kinase, such as protein kinase C, or of tyrosine protein kinase, such as epidermal growth factor receptor tyrosine kinase, a cytokine, a negative growth regulator, such as TGF-β or IFN-β, an aromatase inhibitor, a classical cytostatic, an inhibitor of the interaction of an SH2 domain with a phosphorylated protein, an inhibitor of Bcl-2 and modulators of the Bcl-2 family members such as Bax, Bid, Bad, Bim, Nip3 and BH3-only proteins.

A compound according to the invention is not only for the (prophylactic and preferably therapeutic) management of humans, but also for the treatment of other warm-blooded animals, for example of commercially useful animals, for example rodents, such as mice, rabbits or rats, or guinea-pigs. Such a compound may also be used as a reference standard in the test systems described above to permit a comparison with other compounds.

With the groups of preferred compounds of formula I mentioned hereinafter, definitions of substituents from the general definitions mentioned hereinbefore may reasonably be used, for example, to replace more general definitions with more specific definitions or especially with definitions characterized as being preferred.

In particular, the invention relates to compounds of formula I and salts of such compounds, wherein
R¹ represents hydrogen, lower alkyl, lower alkoxy-lower alkyl or optionally substituted phenyl;
R² represents hydrogen, lower alkyl, lower alkoxy-lower alkyl, optionally substituted phenyl, or R¹ and R² together with the bond connecting them represent a carbocyclic ring;
R³ represents hydrogen or lower alkyl, or R² and R³ together with the bond connecting them represent a carbocyclic ring;
R⁴ and R⁵ represent hydrogen;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl; and
R⁷, R⁸, R⁹ and R¹⁰, independently of each other, represent hydrogen, lower alkyl, halogen, haloalkyl, lower alkoxy, lower alkylthio, lower alkoxy-lower alkoxy, aminoalkoxy wherein the nitrogen atom is unsubstituted or substituted by lower alkyl, hydroxy-lower alkyl or lower alkoxy-lower alkyl or wherein the nitrogen atom is part of heterocyclyl, lower acyloxy, lower alkoxy-lower acyloxy, or di-lower alkylamino-lower acyloxy; or also R⁷ and R⁸, or R⁸ and R⁹, or R⁹ and R¹⁰ together represent methylenedioxy or an annulated aromatic or aliphatic ring; with the proviso that at least one of the substituents R⁷, R⁸, R⁹ and R¹⁰ represents hydrogen;

Preferred are compounds of formula I and salts of such compounds, wherein
R¹ represents hydrogen;
R² represents hydrogen, lower alkyl or optionally substituted phenyl;
R³ represents hydrogen or lower alkyl, or R² and R³ together with the bond connecting them represent a carbocyclic ring;
R⁴ and R⁵ represent hydrogen;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl; and
R⁷, R⁸, R⁹ and R¹⁰, independently of each other, represent hydrogen, lower alkyl, halogen, haloalkyl, lower alkoxy, lower alkylthio, lower alkoxy-lower alkoxy, aminoalkoxy wherein the nitrogen atom is unsubstituted or substituted by lower alkyl, hydroxy-lower alkyl or lower alkoxy-lower alkyl or wherein the nitrogen atom is part of heterocyclyl, lower acyloxy, lower alkoxy-lower acyloxy, or di-lower alkylamino-lower acyloxy; or also R⁷ and R⁸, or R⁸ and R⁹, or R⁹ and R¹⁰ together represent methylenedioxy or an annulated aromatic or aliphatic ring; with the proviso that at least one of the substituents R⁷, R⁸, R⁹ and R¹⁰ represents hydrogen;

Specially preferred are compounds of the formula I and salts of such compounds, wherein
R¹ represents hydrogen;
R² represents hydrogen or lower alkyl;
R³ represents hydrogen or lower alkyl, or R² and R³ together with the bond connecting them represent a carbocyclic ring;
R⁴ and R⁵ represent hydrogen;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl; and
R⁷, R⁸, R⁹ and R¹⁰, independently of each other, represent hydrogen, lower alkyl or lower alkoxy, or R⁸ and R⁹ together represent methylenedioxy, with the proviso that at least one of the substituents R⁷, R⁸, R⁹ and R¹⁰ represents hydrogen.

More preferred are compounds of formula I and pharmaceutically acceptable salts of such compounds, wherein
R¹ represents hydrogen;
R² represents hydrogen or lower alkyl;
R³ represents hydrogen or lower alkyl, or R² and R³ together with the bond connecting them represent a carbocyclic ring;
R⁴ and R⁵ represent hydrogen;
R⁶ represents hydrogen, alkyl, optionally substituted phenyl, halogen, hydroxyalkyl, alkoxyalkyl, or trisubstituted silyl wherein the substituents are lower alkyl or phenyl;
R⁷, R⁸, R⁹ and R¹⁰, independently of each other, represent hydrogen, lower alkyl or lower alkoxy, or R⁸ and R⁹ together represent methylenedioxy, with the proviso that at least one of the substituents R⁷, R⁸, R⁹ and R¹⁰ represents hydrogen.

Particularly preferred are the compounds of the Examples.

Especially, the invention relates to the use of a compound of formula I, a prodrug or a pharmaceutically acceptable salt of such a compound for the preparation of a pharmaceutical composition for the treatment of a neoplastic disease, autoimmune disease, transplantation related pathology and/or degenerative disease.

Furthermore, the invention provides a method for the treatment of a neoplastic disease, autoimmune disease, transplantation related pathology and/or degenerative disease, which comprises administering a compound of formula I, a prodrug or a pharmaceutically acceptable salt thereof, wherein the radicals and symbols have the meanings as defined above, in a quantity effective against said disease, to a warm-blooded animal requiring such treatment.

### Method of preparation

A compound of the invention may be prepared by processes that, though not applied hitherto for the new compounds of the present invention, are known per se, especially a process characterized in that for the synthesis of a compound of the formula I wherein the symbols are as defined, a substituted pyrrolidinedione of formula II 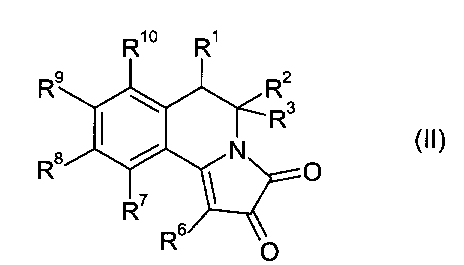 wherein R¹ to R³ and R⁶ to R¹⁰ are as defined for a compound of formula I, is reacted with an optionally substituted o-phenylenediamine of formula III 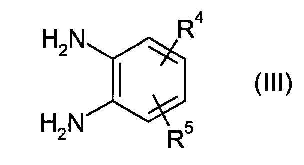 wherein R⁴ and R⁵ are as defined for a compound of formula I, optionally in the presence of a dehydrating agent and an inert base, or an inert acid, and/or a suitable catalyst, and optionally in the presence of an inert solvent; where the above starting compounds of formula II and III may also be present with functional groups in protected form if necessary and/or in the form of salts, provided a salt-forming group is present and the reaction in salt form is possible; any protecting groups in a protected derivative of a compound of the formula I are removed; and, if so desired, an obtainable compound of formula I is converted into another compound of formula I, a free compound of formula I is converted into a salt, an obtainable salt of a compound of formula I is converted into the free compound or another salt, and/or a mixture of isomeric compounds of formula I is separated into the individual isomers.

### Protecting groups

If one or more other functional groups, for example carboxy, hydroxy or amino, are or need to be protected in a compound of formula II or III, because they should not take part in the reaction, these are such groups as are usually used in condensation reactions, e.g. in the synthesis of peptide compounds, also of cephalosporins and penicillins, as well as nucleic acid derivatives and sugars.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter.

The protection of such functional groups by such protecting groups, the protecting groups themselves, and their removal reactions are described for example in standard reference books for peptide synthesis as cited hereinbefore, and in special books on protective groups such as J. F. W. McOmie, "Protective Groups in Organic Chemistry", Plenum Press, London and New York 1973, in "Methoden der organischen Chemie" (Methods of organic chemistry), Houben-Weyl, 4th edition, Volume 15/1, Georg Thieme Verlag, Stuttgart 1974, and in T. W. Greene, "Protective Groups in Organic Synthesis", Wiley, New York.

### Additional process steps

In the additional process steps, carried out as desired, functional groups of the starting compounds which should not take part in the reaction may be present in unprotected form or may be protected for example by one or more of the protecting groups mentioned hereinabove under "protecting groups". The protecting groups are then wholly or partly removed according to one of the methods described there.

In a compound of the formula I wherein R⁶ is halogen, e.g. bromine, iodine or chlorine, this halogen can be exchanged by metals such as lithium or magnesium. This transformation referred to in literature as metal halogen exchange is well known in the art. Suitable reaction conditions include reaction with a metallated alkane such as butyl lithium or ethyl magnesium chloride or a metallated benzene or thiophene in an inert solvent at temperatures of -100°C to room temperature. The resulting metallated derivative of a compound of formula I can be reacted with a broad range of nucleophiles. The overall sequence allows access to compounds of formula I wherein R⁶ represents alkyl, hydroxyalkyl, alkenyl, acyl, halogen, alkylthio etc. Alternatively the metallated derivative of a compound of formula I can be transmetallated before being reacted with a nucleophile allowing modulating the reactivity and thus broadening the scope of this transformation. Important metals introduced by transmetallation include boron, silicon, copper, titanium and nickel. Protecting groups may be necessary to prevent undesired reaction of the metallated derivative with one of the substituents.

Alternatively compounds of formula I wherein R⁶ is halogen, e.g. bromine or iodine, can be coupled with alkines in a reaction known as Sonogashira coupling giving compounds of formula I wherein R⁶ is alkinyl, or with arylboronic acids in a reaction known as Suzuki reaction giving compounds of formula I wherein R⁶ is optionally substituted phenyl or other aromatic group, or under basic conditions with aliphatic or aromatic amines referred to as Buchwald-Hartwig reaction, always in the presence of palladium catalysts.

Compounds of formula I show enamine reactivity. Thus compounds of formula I wherein R⁶ equals hydrogen can be halogenated allowing access to compounds of formula I wherein R⁶ is halogen useful in metal-halogen exchange reactions described hereinbefore. Catalyzed or uncatalyzed reaction of compounds of formula I wherein R⁶ is hydrogen with activated carboxylic acid derivatives such as acyl halogenides or acid anhydrides allow the introduction of a corresponding acyl group R⁶ in a Friedel-Crafts type acylation. Other important transformations of compounds of formula wherein R⁶ is hydrogen include formylation, cyanogenation, aminoalkylation, nitration, sulfenylation, sulfonylation, chlorosulfonylation and the like. These transformations can be performed under conditions typically used for such reactions. Thus formylation is achieved using a formyl cation equivalent prepared for example by reaction of phosphoroxy chloride and a N,N-dialkylformamide. Similarly under Mannich-reaction conditions the introduction of aminoalkyl groups is possible.

Salts of a compound of formula I with a salt-forming group may be prepared in a manner known per se. Acid addition salts of compounds of formula I may thus be obtained by treatment with an acid or with a suitable anion exchange reagent. Salts can usually be converted to free compounds, e.g. by treating with suitable basic agents, for example with alkali metal carbonates, alkali metal hydrogencarbonates, or alkali metal hydroxides, typically potassium carbonate or sodium hydroxide.

Stereoisomeric mixtures, e.g. mixtures of diastereomers, can be separated into their corresponding isomers in a manner known per se by means of suitable separation methods. Diastereomeric mixtures for example may be separated into their individual diastereomers by means of fractionated crystallization, chromatography, solvent distribution, and similar procedures. This separation may take place either at the level of a starting compound or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

In a compound of the formula I wherein in a group R⁶, R⁷, R⁸, R⁹ and/or R¹⁰ hydrogen is attached to a nitrogen or oxygen atom and should be converted to the respective compound wherein hydrogen is replaced by lower alkyl, this may be performed by reaction e.g. with a diazo lower alkyl compound, especially diazomethane, in an inert solvent, preferably in the presence of copper, copper(I)-chloride or copper(11)-sulfate. Also reaction with lower alkylhalogenides is possible, or with other leaving group carrying lower alkanes, e.g. lower alkyl alcohols esterified by a strong organic sulfonic acid, e.g methanesulfonic acid or p-toluene sulfonic acid.

### Intermediates

Compounds of formula II can be prepared according to known methods by ring closure of suitably substituted dihydroisoquinolines IV with oxalyl chloride following the procedure described by A.G. Mikhailovsky, V.S. Shklyaev, Khim. Geterotsikl. Soedin 1994, (7), 946-949.

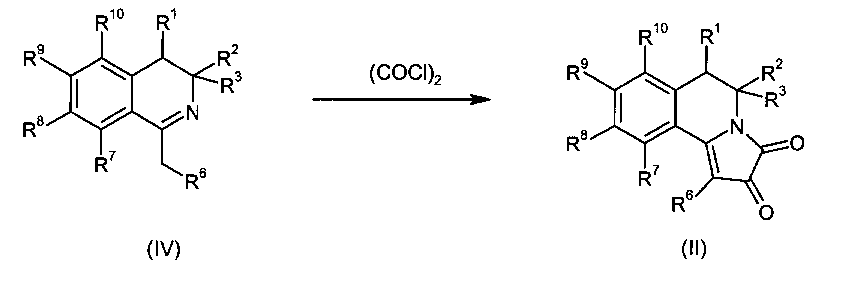

Compounds of formula IV can be prepared by cyclization of an alcohol of formula V with a nitrile of formula VI. Lewis acids are needed to promote this reaction. The substituent X in the nitrile of formula VI represents hydrogen or a protecting group such as alkoxycarbonyl. Depending on the reaction conditions this group is cleaved or retained in the dihydroisoquinoline of formula IV. If desired the protecting group X can be cleaved in a separate step.

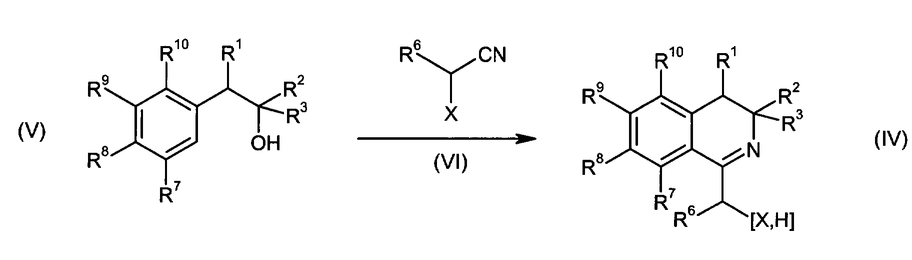

Alternatively compounds of formula IV can be prepared by direct cyclization of a benzene derivative of formula VII with an epoxide of formula VIII and a nitrile of formula VI. This reaction is promoted by Lewis acids and is limited to compounds of formula VII wherein at least one of the substituents R⁷ to R¹⁰ is an electron donating group such as alkoxy or alkylthio.

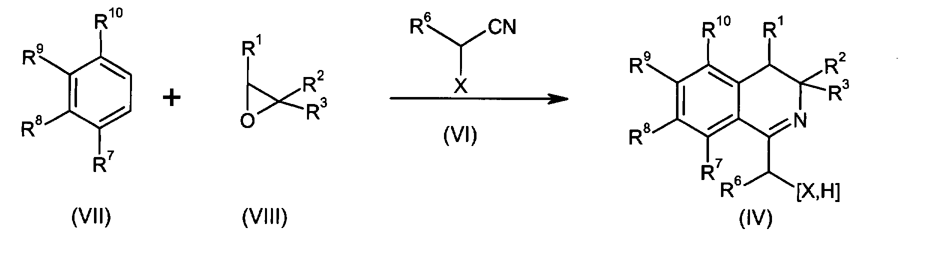

Compounds of formula III are either commercially available or can be prepared according to procedures well known in the art.

### General process conditions

All process steps described here can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralising agents, for example ion exchangers, typically cation exchangers, for example in the H⁺ form, depending on the type of reaction and/or reactants at reduced, normal, or elevated temperature, for example in the range from -100°C to about 190°C, preferably from about -80°C to about 150°C, for example at -80 to -60°C, at room temperature, at - 20 to 40°C or at the boiling point of the solvent used, under atmospheric pressure or in a closed vessel, where appropriate under pressure, and/or in an inert atmosphere, for example under argon or nitrogen.

Salts may be present in all starting compounds and transients, if these contain salt-forming groups. Salts may also be present during the reaction of such compounds, provided the reaction is not thereby disturbed. At all reaction stages, isomeric mixtures that occur can be separated into their individual isomers, e.g. diastereomers or enantiomers, or into any mixtures of isomers, e.g. racemates or diastereomeric mixtures.

The invention relates also to those forms of the process in which one starts from a compound obtainable at any stage as a transient and carries out the missing steps, or breaks off the process at any stage, or forms a starting material under the reaction conditions, or uses said starting material in the form of a reactive derivative or salt, or produces a compound obtainable by means of the process according to the invention and processes the said compound in situ. In the preferred embodiment, one starts from those starting materials which lead to the compounds described hereinabove as preferred.

In the preferred embodiment, a compound of formula I is prepared according to or in analogy to the processes and process steps defined in the Examples.

The compounds of formula I, including their salts, are also obtainable in the form of hydrates, or their crystals can include for example the solvent used for crystallization (present as solvates).

### Pharmaceutical preparations, methods, and uses

The present invention relates also to pharmaceutical compositions that comprise a compound of formula I as active ingredient and that can be used especially in the treatment of the diseases mentioned at the beginning. Compositions for enteral administration, such as nasal, buccal, rectal or, especially, oral administration, and for parenteral administration, such as intravenous, intramuscular or subcutaneous administration, to warm-blooded animals, especially humans, are especially preferred. The compositions comprise the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The present invention relates especially to pharmaceutical compositions that comprise a compound of formula I, a tautomers, a prodrug or a pharmaceutically acceptable salt, or a hydrate or solvate thereof, and at least one pharmaceutically acceptable carrier.

The invention relates also to pharmaceutical compositions for use in a method for the prophylactic or especially therapeutic management of the human or animal body, in particular in a method of treating neoplastic disease, autoimmune disease, transplantation related pathology and/or degenerative disease, especially those mentioned hereinabove.

The invention relates also to processes and to the use of compounds of formula I thereof for the preparation of pharmaceutical preparations which comprise compounds of formula I as active component (active ingredient).

A pharmaceutical composition for the prophylactic or especially therapeutic management of a neoplastic disease, autoimmune disease, transplantation related pathology and/or degenerative disease, of a warm-blooded animal, especially a human or a commercially useful mammal requiring such treatment, comprising a novel compound of formula I as active ingredient in a quantity that is prophylactically or especially therapeutically active against the said diseases, is likewise preferred.

The pharmaceutical compositions comprise from approximately 1 % to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories, or capsules. Further dosage forms are, for example, ointments, creams, pastes, foams, tinctures, lip-sticks, drops, sprays, dispersions, etc. Examples are capsules containing from about 0.05 g to about 1.0 g active ingredient.

The pharmaceutical compositions of the present invention are prepared in a manner known per se, for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredient alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known per se, for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80® (polyoxyethylene(20)sorbitan mono-oleate).

Suspensions in oil comprise as the oil component the vegetable, synthetic, or semi-synthetic oils customary for injection purposes. In respect of such, special mention may be made of liquid fatty acid esters that contain as the acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brassidic acid or linoleic acid, if desired with the addition of antioxidants, for example vitamin E, β-carotene or 3,5-di-tert-butyl-4-hydroxytoluene. The alcohol component of these fatty acid esters has a maximum of 6 carbon atoms and is a monovalent or polyvalent, for example a mono-, di- or trivalent, alcohol, for example methanol, ethanol, propanol, butanol or pentanol or the isomers thereof, but especially glycol and glycerol. As fatty acid esters, therefore, the following are mentioned: ethyl oleate, isopropyl myristate, isopropyl palmitate, "Labrafil M 2375" (polyoxyethylene glycerol), "Labrafil M 1944 CS" (unsaturated polyglycolized glycerides prepared by alcoholysis of apricot kernel oil and consisting of glycerides and polyethylene glycol ester), "Labrasol" (saturated polyglycolized glycerides prepared by alcoholysis of TCM and consisting of glycerides and polyethylene glycol ester; all available from Gattefossé, France), and/or "Miglyol 812" (triglyceride of saturated fatty acids of chain length C₈ to C₁₂ from Hüls AG, Germany), but especially vegetable oils such as cottonseed oil, almond oil, olive oil, castor oil, sesame oil, soybean oil and more especially groundnut oil.

The manufacture of injectable preparations is usually carried out under sterile conditions, as is the filling, for example, into ampoules or vials, and the sealing of the containers. Pharmaceutical compositions for oral administration can be obtained, for example, by combining the active ingredient with one or more solid carriers, if desired granulating a resulting mixture, and processing the mixture or granules, if desired or necessary, by the inclusion of additional excipients, to form tablets or tablet cores.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

Pharmaceutical compositions for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or microbicides, such as sorbic acid or benzoic acid.

The present invention relates furthermore to a method for the treatment of a neoplastic disease, autoimmune disease, transplantation related pathology and/or degenerative disease, which comprises administering a compound of formula I or a pharmaceutically acceptable salt thereof, wherein the radicals and symbols have the meanings as defined above for formula I, in a quantity effective against said disease, to a warm-blooded animal requiring such treatment. The compounds of formula I can be administered as such or especially in the form of pharmaceutical compositions, prophylactically or therapeutically, preferably in an amount effective against the said diseases, to a warm-blooded animal, for example a human, requiring such treatment. In the case of an individual having a bodyweight of about 70 kg the daily dose administered is from approximately 0.05 g to approximately 5 g, preferably from approximately 0.25 g to approximately 1.5 g, of a compound of the present invention.

The present invention relates especially also to the use of a compound of formula I, or a pharmaceutically acceptable salt thereof, especially a compound of formula I which is said to be preferred, or a pharmaceutically acceptable salt thereof, as such or in the form of a pharmaceutical formulation with at least one pharmaceutically acceptable carrier for the therapeutic and also prophylactic management of one or more of the diseases mentioned hereinabove, in particular a neoplastic disease, autoimmune disease, transplantation related pathology and/or degenerative disease.

The preferred dose quantity, composition, and preparation of pharmaceutical formulations (medicines) which are to be used in each case are described above.

The following Examples serve to illustrate the invention without limiting the invention in its scope.

### Examples

Synthesis of compounds of the invention:

### Example 1: 6,6-Dimethyl-5,6-dihydro-6a,7,12-triaza-dibenzo[a,h]fluorene (1)

A mixture of 5,5-dimethyl-5,6-dihydro-pyrrolo[2,1a]isoquinoline-2,3-dione (550 mg, 2.42 mmol) and o-phenylenediamine (261 mg, 2.42 mmol) in acetic acid (5 ml) is refluxed for 2 h. The reaction mixture is diluted with water, extracted with ether, washed with brine, dried over Na₂SO₄ and concentrated. Purification of the crude product by chromatography (5% ethyl acetate/hexanes) affords the title compound, mp 124-125°C. ¹H-NMR (CDCl₃, 400 MHz): 8.28-8.25 (m,1H); 8.22-8.19 (m,1H); 8.05-8.04 (m,1H); 7.76-7.74 (m,2H); 7.52-7.49 (m,2H); 7.39 (m,1H); 7.24 (s,1H); 3.26 (s,2H); 2.02 (s,6H).

### A) 5,5-Dimethyl-5,6-dihydro-pyrrolo[2,1a]isoquinoline-2,3-dione

To a solution of oxalyl chloride (0.86 ml, 10 mmol) in anhydrous ether (50 ml) is added a solution of 1,1,3-trimethyl-3,4-dihydroisoquinoline (2.09 g, 10 mmol) in triethylamine (2.76 ml, 20 mmol) and ether (100 ml) within 15 minutes under nitrogen at 0°C. The mixture is stirred for additional 2 h. The reaction mixture is diluted with water, extracted with ether, washed with brine, dried over Na₂SO₄ and concentrated. Purification of the crude product by chromatography (20% ethyl acetate/hexanes) affords the title compound, mp 122-124°C.

### B) 1,1,3-Trimethyl-3,4-dihydroisoquinoline

To a solution of ethyl cyanoacetate (4.14 g, 33.3 mmol) in dry benzene (85 ml) is added concentrated sulfuric acid keeping the temperature below 5°C. After 30 minutes a solution of 2-methyl-1 -phenyl-propan-2-ol in benzene (55 ml) is added over a period of 30 minutes. The reaction mixture is refluxed for 2.5 h and poured onto ice. The organic layer is separated and neutralized with concentrated ammonia, extracted with ether, dried and concentrated. Purification by chromatography (40% ethyl acetate/hexanes) gives the title compound as light yellow oil.

### Example 2: 13-Bromo-6,6-dimethyl-5,6-dihydro-6a,7,12-triaza-dibenzo[a,h]fluorene (2)

To a solution of 6,6-dimethyl-5,6-dihydro-6a,7,12-triaza-dibenzo[a,h]fluorene (1) (60 mg, 0.2 mmol) in acetonitrile (5 ml) is added N-bromosuccinimide (71.2 mg, 0.2 mmol) at 0°C. After stirring for 3 h the solvent is evaporated under reduced pressure and the residue is purified by chromatography (10% ethyl acetate/hexanes) to afford the title compound as a yellow solid of mp 165-167°C.

### Example 3: 6,6-Dimethyl-5,6-dihydro-6a,7,12-triaza-dibenzo[a,h]fluorene-13-carbaldehyde (3)

To a solution of 13-bromo-6,6-dimethy)-5,6-dihydro-6a,7,12-triaza-dibenzo[a,h]f)uorene (2) (100 mg, 0.26 mmol) in dry THF n-BuLi (1.6 M, 0.18 ml, 0.29 mmol) is added at -78°C under nitrogen with stirring. The mixture is stirred for 1 h before adding dry N,N-dimethylformamide (28 mg, 0.39 mmol). The reaction mixture is allowed to reach room temperature over a period of 20 minutes. It is then quenched by adding 1 M HCI (1 ml) and extracted repeatedly with chloroform. The combined organic layer is dired over Na₂SO₄ and concentrated. The crude product is purified by chromatography (20% ethyl acetate/hexanes) to yield the title compound, mp 194-196°C.

### Example 4: 1-(6,6-Dimethyl-5,6-dihydro-6a,7,12-triaza-dibenzo[a,h]fluoren-13-yl)-2,2,2-trifluoro-ethanone (4)

Trifluoroacetic anhydride (70 mg, 0.32 mmol) is added to a solution of 6,6-dimethyl-5,6-dihydro-6a,7,12-triaza-dibenzo[a,h]fluorene (1) (50 mg, 0.16 mmol) in dry dichloromethane (4 ml) and triethylamine (66 mg, 0.66 mmol) at 0°C under an atmosphere of nitrogen. The reaction mixture is allowed to reach room temperature and is stirred overnight. After quenching with water the product is extracted with ethyl acetate. The extract is dried over Na₂SO₄and concentrated to yield the product. Purification by flash chromatography yields the title compound, mp 128-132°C.

### Example 5: 2-Hydroxy-3-methoxy-6,6-dimethyl-5 6-dihydro-6a,7,12-triazadibenzo[a,h]fluorene (5)

A mixture of 9-(t-butyl-dimethyl-silyloxy)-8-methoxy-5,5-dimethyl-5,6-dihydro-pyrrolo[2,1a] isoquinoline-2,3-dione (150 mg, 0.39 mmol) and o-phenylenediamine (42 mg, 0.39 mmol) in acetic acid (2 ml) is refluxed for 2 h. The reaction mixture is diluted with water, extracted with ether, washed with brine, dried over Na₂SO₄and concentrated. Purification of the crude product by chromatography (20% ethyl acetate/hexanes) affords the title compound, mp 124-125°C. ¹H-NMR (CDCl₃, 400 MHz): 8.14 (m,1H); 8.04 (m,1H); 7.61 (m,2H); 7.47 (s,1H); 6.97 (s,1H); 6.74 (s,1H); 3.95 (s,3H); 3.08 (s,2H); 1.92 (s,6H).

### A) 9-(t-Butyl-dimethyl-silyloxy)-8-methoxy-5,5-dimethyl-5,6-dihydro-pyrrolo[2,1a]isoquinoline-2,3-dione

To a solution of 6-methoxy-7-(t-butyl-dimethyl-silyloxy)-1,3,3-trimethyl-3,4-dihydroisoquinoline (300 mg, 0.9 mmol) in dichloromethane (5 ml) is added sequentially triethylamine (0.25 ml, 1.8 mmol) and a solution of oxalyl chloride (0.086 ml, 1 mmol) in dichloromethane (10 ml) under nitrogen at 0°C. The mixture is stirred for additional 2 h at room temperature. The reaction mixture is diluted with water, extracted with ether, washed with brine, dried over Na₂SO₄ and concentrated. Purification of the crude product by chromatography (10% ethyl acetate / hexanes) affords the title compound. ¹H-NMR (CDCl₃, 400 MHz): 7.17 (s, 1H); 6.67 (s,1H); 5.62 (s,1H); 3.88 (s,3H); 2.87 (s,2H); 1.58 (s,6H); 0.98 (s,9H); 0.15 (s,6H).

### Example 6: [2-(3-Methoxy-6,6-dimethyl-5,6-dihydro-6a,7,12-triaza-dibenzofluoren-2-yloxy)-ethyl]-dimethyl-amine (6)

A mixture of 2-hydroxy-3-methoxy-6,6-dimethyl-5,6-dihydro-6a,7,12-triaza-dibenzo[a,h] fluorene (5) (100 mg, 0.29 mmol), N,N-dimethylaminoethylchloride hydrochloride (62.6 mg, 0.43 mmol) in acetone (5 ml) is refluxed in the presence of a catalytic amount of tetrabutylammonium iodide for 16 h. The resulting mixture is cooled and filtered. Concentration of the filtrate under reduced pressure and purification by chromatography (10% methanol / dichloromethane) yields the title compound, mp 135-137°C. The following compounds were prepared in an analogous manner:

**(Table 1)**

| No. | R¹ | R² | R³ | R⁴ | R⁵ | R⁶ | R⁷ | R⁸ | R⁹ | R¹⁰ | mp | salt |
|---|---|---|---|---|---|---|---|---|---|---|---|---|
| 7 | H | H | Me | H | H | H | H | OAc | OMe | H | 135-137- | |
| 8 | H | H | Me | H | H | H | H | OMe | OMe | H | 120-122- | |
| 9 | H | H | Me | H | H | H | H | OMe | OMe | H | 216-217 | HCl |
| 10 | H | H | Me | H | H | Br | H | OMe | OMe | H | 192-194- | |
| 11 | H | H | Me | H | H | Cl | H | H | H | H | 160-162 - | |
| 12 | H | H | Me | H | H | CI | H | OMe | OMe | H | 218-220 - | |
| 13 | H | H | Et | H | H | H | H | OMe | OMe | H | 157-160- | |
| 14 | H | H | Et | H | H | H | H | OMe | OMe | H | 205-208 | HCI |
| 15 | H | H | Me | H | H | H | H | H | H | H | 220-224 | HCI |
| 16 | H | H | Me | H | H | H | H | OCH₂OMe | OMe | H | 134-136- | |

Likewise the following compounds (R² = H) can be prepared:

**(Table 2)**

| No. | R¹ | R³ | R⁴ | R⁵ | R⁶ | R⁷ | R⁸ | R⁹ | R¹⁰ |
|---|---|---|---|---|---|---|---|---|---|
| 17 | Me | Me | H | H | H | H | H | H | H |
| 18 | H | Me | H | H | t-Bu | H | H | H | H |
| 19 | H | Me | H | H | SiMe₃ | H | H | H | H |
| 20 | H | Me | H | H | CH₂OMe | H | H | H | H |
| 21 | H | Me | H | H | CHOHMe | H | H | H | H |
| 22 | H | Me | H | H | CH₂Ph | H | H | H | H |
| 23 | H | Me | H | H | CH₂NMe₂ | H | H | H | H |
| 24 | H | Me | H | H | CH₂NEt₂ | H | H | H | H |
| 25 | H | Me | H | H | CHOHPh | H | H | H | H |
| 26 | H | Me | H | H | Ph | H | H | H | H |
| 27 | H | Me | H | H | m-CI-Ph | H | H | H | H |
| 28 | H | Me | H | H | o-CI-Ph | H | H | H | H |
| 29 | H | Me | H | H | C≡CH | H | H | H | H |
| 30 | H | Me | H | H | C≡C-CH₂OH | H | H | H | H |
| 31 | H | Me | H | H | C≡C-CH₂NMe₂ | H | H | H | H |
| 32 | H | Me | H | H | Me | H | H | H | H |
| 33 | H | Me | H | H | Et | H | H | H | H |
| 34 | H | Me | H | H | Pr | H | H | H | H |
| 35 | H | Me | H | H | i-Pr | H | H | H | H |
| 36 | H | Me | H | H | COOMe | H | H | H | H |
| 37 | H | Me | H | H | CONHPh | H | H | H | H |
| 38 | H | Me | H | H | SO₂CF₃ | H | H | H | H |
| 39 | H | Me | H | H | SOCF₃ | H | H | H | H |
| 40 | H | Me | H | H | COOH | H | H | H | H |
| 41 | H | Me | H | H | m-F-Ph | H | H | H | H |
| 42 | H | Me | H | H | t-Bu | H | OCH₂OMe | OMe | H |
| 43 | H | Me | H | H | SiMe₃ | H | OMe | OMe | H |
| 44 | H | Me | H | H | CH₂OMe | H | OMe | OMe | H |
| 45 | H | Me | H | H | CHOHMe | H | OMe | OMe | H |
| 46 | H | Me | H | H | CH₂Ph | H | OMe | OMe | H |
| 47 | H | Me | H | H | CH₂NMe₂ | H | OMe | OMe | H |
| 48 | H | Me | H | H | CH₂NEt₂ | H | OCH₂OMe | OMe | H |
| 49 | H | Me | H | H | CHOHPh | H | OMe | OMe | H |
| 50 | H | Me | H | H | Ph | H | OMe | OMe | H |
| 51 | H | Me | H | H | m-CI-Ph | H | OMe | OMe | H |
| 52 | H | Me | H | H | o-CI-Ph | H | OMe | OMe | H |
| 53 | H | Me | H | H | C≡CH | H | OMe | OMe | H |
| 54 | H | Me | H | H | C≡C-CH₂OH | H | OMe | OMe | H |
| 55 | H | Me | H | H | C≡C-CH₂NMe₂ | H | OMe | OMe | H |
| 56 | H | Me | H | H | Me | H | OMe | OMe | H |
| 57 | H | Me | H | H | Et | H | OCH₂OMe | OMe | H |
| 58 | H | Me | H | H | Pr | H | OMe | OMe | H |
| 59 | H | Me | H | H | i-Pr | H | OCH₂OMe | OMe | H |
| 60 | H | Me | H | H | COOMe | H | OMe | OMe | H |
| 61 | H | Me | H | H | CONHPh | H | OMe | OMe | H |
| 62 | H | Me | H | H | SO₂CF₃ | H | OMe | OMe | H |
| 63 | H | Me | H | H | SOCF₃ | H | OMe | OMe | H |
| 64 | H | Me | H | H | COOH | H | OMe | OMe | H |
| 65 | H | Me | H | H | m-F-Ph | H | OMe | OMe | H |
| 66 | H | Me | H | H | CN | H | OMe | OMe | H |
| 67 | H | Me | H | H | C≡C-t-Bu | H | OMe | OMe | H |
| 68 | H | Me | H | H | C≡C-Ph | H | OMe | OMe | H |
| 69 | H | Me | H | H | C≡C-t-Bu | H | OMe | OMe | H |
| 70 | H | Me | H | H | SPh | H | OMe | OMe | H |
| 71 | H | Me | H | H | Br | OMe | H | OMe | H |
| 72 | H | Me | H | H | Cl | OMe | H | OMe | H |
| 73 | H | Me | H | H | t-Bu | OMe | H | OMe | H |
| 74 | H | Me | H | H | SiMe₃ | OMe | H | OMe | H |
| 75 | H | Me | H | H | CH₂OMe | OMe | H | OMe | H |
| 76 | H | Me | H | H | CHOHMe | OMe | H | OMe | H |
| 77 | H | Me | H | H | CH₂Ph | OMe | H | OMe | H |
| 78 | H | Me | H | H | CH₂NMe₂ | OMe | H | OMe | H |
| 79 | H | Me | H | H | CH₂NEt₂ | OMe | H | OMe | H |
| 80 | H | Me | H | H | CHOHPh | OMe | H | OMe | H |
| 81 | H | Me | H | H | Ph | OMe | H | OMe | H |
| 82 | H | Me | H | H | m-CI-Ph | OMe | H | OMe | H |
| 83 | H | Me | H | H | o-CI-Ph | OMe | H | OMe | H |
| 84 | H | Me | H | H | C≡CH | OMe | H | OMe | H |
| 85 | H | Me | H | H | C≡C-CH₂OH | OMe | H | OMe | H |
| 86 | H | Me | H | H | C≡C-CH₂NMe₂ | OMe | H | OMe | H |
| 87 | H | Me | H | H | Me | OMe | H | OMe | H |
| 88 | H | Me | H | H | Et | OMe | H | OMe | H |
| 89 | H | Me | H | H | Pr | OMe | H | OMe | H |
| 90 | H | Me | H | H | i-Pr | OMe | H | OMe | H |
| 91 | H | Me | H | H | COOMe | OMe | H | OMe | H |
| 92 | H | Me | H | H | CONHPh | OMe | H | OMe | H |
| 93 | H | Me | H | H | SO₂CF₃ | OMe | H | OMe | H |
| 94 | H | Me | H | H | SOCF₃ | OMe | H | OMe | H |
| 95 | H | Me | H | H | COOH | OMe | H | OMe | H |
| 96 | H | Me | H | H | m-F-Ph | OMe | H | OMe | H |
| 97 | H | Me | H | H | CN | OMe | H | OMe | H |
| 98 | H | Me | H | H | C≡C-t-Bu | OMe | H | OMe | H |
| 99 | H | Me | H | H | C≡C-Ph | OMe | H | OMe | H |
| 100 | H | Me | H | H | C≡C-t-Bu | OMe | H | OMe | H |
| 101 | H | Me | H | H | SPh | OMe | H | OMe | H |
| 102 | H | Me | H | H | Br | H | OCH₂O | | H |
| 103 | H | Me | H | H | CH₂OMe | H | OCH₂O | | H |
| 104 | H | Me | H | H | CHOHMe | H | OCH₂O | | H |
| 105 | H | Me | H | H | CH₂Ph | H | OCH₂O | | H |
| 106 | H | Me | H | H | CH₂NMe₂ | H | OCH₂O | | H |
| 107 | H | Me | H | H | CH₂NEt₂ | H | OCH₂O | | H |
| 108 | H | Me | H | H | CHOHPh | H | OCH₂O | | H |
| 109 | H | Me | H | H | Ph | H | OCH₂O | | H |
| 110 | H | Me | H | H | m-CI-Ph | H | OCH₂O | | H |
| 111 | H | Me | H | H | o-CI-Ph | H | OCH₂O | | H |
| 112 | H | Me | H | H | Me | H | OCH₂O | | H |
| 113 | H | Me | H | H | Et | H | OCH₂O | | H |
| 114 | H | Me | H | H | Pr | H | OCH₂O | | H |
| 115 | H | Me | H | H | i-Pr | H | OCH₂O | | H |
| 116 | H | Me | H | H | COOMe | H | OCH₂O | | H |
| 117 | H | Me | H | H | CONHPh | H | OCH₂O | | H |
| 118 | H | Me | H | H | SO₂CF₃ | H | OCH₂O | | H |
| 119 | H | Me | H | H | SOCF₃ | H | OCH₂O | | H |
| 120 | H | Me | H | H | COOH | H | OCH₂O | | H |
| 121 | H | Me | H | H | m-F-Ph | H | OCH₂O | | H |

Similarly, the following compounds can be prepared:
Compounds No. 122-242, wherein substituents R¹ and R³ to R¹⁰ are as in compounds No. 1-121 (Examples 1 to 6, Table 1 and Table 2), but R² equals phenyl instead of hydrogen.
Compounds No. 243-363, wherein substituents R¹ and R³ to R¹⁰ are as in compounds No. 1-121 (Examples 1 to 6, Table 1 and Table 2), but R² equals t-butyl instead of hydrogen.
Compounds No. 364-484, wherein substituents R¹ and R³ to R¹⁰ are as in compounds No. 1-121 (Examples 1 to 6, Table 1 and Table 2), but R² equals isopropyl instead of hydrogen.
Compounds No. 485-605, wherein substituents R¹ and R³ to R¹⁰ are as in compounds No. 1-121 (Examples 1 to 6, Table 1 and Table 2), but R² equals 3-methoxypropyl instead of hydrogen.

General methods for testing of compounds of the invention:

### Example 7: Cell cultures and cell lines.

Cell lines are cultured in RPMI-1640 tissue culture medium containing either 5% or 10% fetal calf serum, 0.05 mM 2-mercaptoethanol, 2 mM glutamine and penicillin/streptomycin 50 µg/ml (complete medium) (Sigma, Buchs, Switzerland). General growth conditions are 37°C and 7.5% CO₂.

The following mouse cell lines (either EGFP transfected or not) are being used: A20.2J (ATCC: TIB-208), MC57G (ATCC: CRL-2295).

The following human cell lines (either EGFP transfected or not) are being used: HeLa (ATCC: CCL-2), KB (ATCC: CCL-17), MCF7 (ATCC: HTB-22), SK-BR-3 (ATCC: HTB-30), SK-Mel 1 (ATCC: HTB-67), SK-Mel 28 (ATCC: HTB-72), PC-3 (ATCC: CRL-1435), SW 480 (ATCC: CCL-228), NCI-H460 (ATCC: HTB-177), NCI-H1792 (ATCC: CRL-5895), HT1080 (ATCC: CCL-21), Jurkat (ATCC: TIB-152), Ramos (ATCC: CRL-1596), Raji (ATCC: CCL-86), H9 (ATCC: HTB-176), Hut78 (ATCC: TIB-161 ), K562 (ATCC: CCL 243), HL-60 (ATCC: CCL 240), U-87MG (ATCC: HTB-14), HepG2 (ATCC: HB-8065), U-2 OS (ATCC: HTB-96), Saos-2 (ATCC: HTB-85), U937 (ATCC: CRL 1593), Hs 578T (ATCC: HTB 126), HBL-100 (ATCC: HTB 124), Molt-4 (ATCC: CRL 1582).

### Example 8: Primary screening setup

All the manipulations are performed under sterile conditions. The assays are being performed in commercially available 96 or 384 well flat bottom clear microtiter plates (Greiner, Germany) respectively, which are suitable for tissue culture techniques.
A defined number of EGFP transfected adherent test cells (96 well plates: 10⁴ - 10⁵, 384 well plates: 1500 - 2*10⁴) are plated out 24 h before treatment either in 75 µl (96 well plates) or 60 µl (384 well plates) complete medium per well in order to ensure appropriate cell spreading. For this purpose a peristaltic pump (e.g. Multidrop by Thermo-Labsystems, Finland) or another suitable device is used. Cells in suspension are plated out according to the same procedure but 1 h prior to treatment. Between seeding out and treatment or addition of compounds the cells are incubated at 37°C under 7.5% CO₂. Subsequently, the compounds under investigation are added at defined concentrations (40 - 80 µM in either 25 µl (96 well plates) or 20 µl (384 well plates) complete medium containing max 4% DMSO) with an appropriate device (e.g. liquid handling system, multi channel pipette etc.) resulting in a final concentration in the test well of 10 - 20 µM compound in max 1% DMSO. Immediately after the addition of the compounds to the cells the zero fluorescence value (t = 0 h) is determined by using a fluorescence microplate reader in order to be able to normalize the fluorescence activities. Afterwards, the test plates are further incubated for a total of 48 h at 37°C under 7.5% CO₂ and are shortly removed only for the purpose of measurement at 8 h, 24 h and 48 h, respectively.

### Example 9: Measurement and quantification of the primary screening.

Relative fluorescence activities of EGFP in compound treated test cells in relation to control cells and cells treated with standard drugs are measured by using a BMG Fluostar microplate fluorescence reader equipped with a filter pair for excitation/emission at 485 nm / 520 nm. The optimum signal to noise ratio is detected by using the time-resolved mode of measurement with a delay of 20 µs and an integration time over 1 ms. The gain is adjusted in such a way that the control cells produce a fluorescence activity of 90% of the maximum. Kinetics is performed by measuring the relative fluorescence activities at t = 0 h, 8 h, 24 h and 48 h. Crude fluorescence activities are individually normalized for different cell numbers and various optical activities of the test compounds / plate-wells by dividing each value from t = 8 h, 24 h and 48 h by the value of t = 0 h resulting in E(8), E(24) and E(48) values. Subsequently, the E(x) values are further processed by forming the inverse (Q-value) of the products E(8)*E(24)*E(48) which result in numbers > 1 for apoptotic / necrotic activities of the compounds and numbers < 1 for proliferative activities of the compounds. Controls (untreated) show values similar to 1. Compounds producing Q values > 2 are being considered relevant in terms of apoptotic / necrotic activity and are subsequently tested in the secondary screening setup.

### Example 10: Secondary screening setup.

All the manipulations are performed under sterile conditions. The assays are being performed in case of adherent cells in commercially available 24 well flat bottom tissue culture plates (Greiner, Germany) and in case of suspension cells in polypropylene tubes (P-tubes) 1.4 ml (Matrix, UK), respectively.
Adherent test cells: 2*10⁴- 4*10⁴ of EGFP transfected cells in 0.5 ml complete medium are plated out 24 h before treatment. At t = 0 the medium is removed and 450 µl new complete medium is added. Subsequently, 50 µl complete medium containing the test compound in max. 5% DMSO is added resulting in final concentrations of 20 µM, 10 µM, 3 µM,1 µM and 0.3 µM of the test compounds, respectively. After 48 h incubation the cells are harvested and analyzed with fluorescence activated cell scanning device (FACSCalibur™, BD Biosciences) according to standard procedures.
Suspension cells: 10⁵ test cells in 450 µl complete medium are pipetted into P-tubes. 50 µl complete medium containing the compounds (see adherent cells) is added immediately. After 48 h of incubation the test cells are analyzed directly on a FACSCalibur™.

### Example 11: Quantification of the secondary screening.

By monitoring the EGFP fluorescence activity in FL1 on a FACSCalibur™, it is possible to distinguish between proliferating cells, apoptotic cells and necrotic cells within the same cell population. The proliferating cells show a high GFP fluorescence activity, the apoptotic population shows an intermediate fluorescence activity whereas the necrotic cells demonstrate a residual fluorescence activity comparable to mock-transfected cells. Within the CellQuest Software (BD Biosciences) three regions are defined in the histogram: M1 comprising the proliferating cells, M2 comprising the apoptotic cell population and M3 comprising the necrotic cell population. As readout the relative abundance of the cells belonging either to M1, M2 or M3 are expressed. Compounds inducing M2 values > 50% and M3 values < 30% are being considered relevant and are further tested and characterized in the tertiary / advanced screening setup.

### Example 12: Tertiary screening setup

### A) Hoechst 33342 nuclear staining

This assay is performed in 96 well tissue culture plates. Appropriate number of cells (adherent cells: 3 - 5*10³, suspension cells: 8 - 10*10³) are being seeded out in 80 µl complete medium. Adherent cells are incubated for 24 h for proper spreading out before addition of test compounds while suspension cells are immediately treated with test compounds after seeding out. The test compounds are added in 20 µl complete medium containing max 5% DMSO. The final compound concentrations in the assays are 10 µM, 3 µM,1 µM and 0.3 µM, respectively. After 24 h or 48 h incubation at culture conditions, 10 µl medium containing Hoechst 33342 dye (Sigma B-2261) at 2-5 µg/ml are added to each well. The assay plates are then further incubated for 30 minutes and subsequently analyzed with a standard inverted fluorescence microscope.
The readout allows the determination of the fraction of apoptotic nuclei as well as other morphological criteria specific for apoptosis as a function of the treatment. Results are indicated in Table 3. The following scores are used: 0 relating to no activity, 1 relating to weak activity comprising less than 50% of the cells and score 2 relating to strong activity comprising more than 50% of the cells.

**Table 3:**

| Hoechst 33342 nuclear staining | | | | | | | | | | | | | |
|---|---|---|---|---|---|---|---|---|---|---|---|---|---|
| No. | conc | KB | | HeLa | | Jurkat | | A20 | | K562 | | Ramos | |
| | | 24h | 48h | 24h | 48h | 24h | 48h | 24h | 48h | 24h | 48h | 24h | 48h |
| 1 | 10 | 0 | 0 | 0 | 2 | 2 | 2 | | | nd | 2 | | |
| | 3 | 0 | 0 | 0 | 2 | 2 | 2 | | | 0 | 2 | | |
| | 1 | 0 | 0 | 0 | 2 | 1 | 2 | | | 0 | 1 | | |
| | 0.3 | 0 | 0 | 0 | 0 | 1 | 2 | | | 0 | 0 | | |
| 2 | 10 | 0 | 0 | 0 | 2 | 2 | 2 | | | nd | 2 | 2 | 2 |
| | 3 | 0 | 0 | 0 | 2 | 2 | 2 | | | 1 | 2 | 0 | 2 |
| | 1 | 0 | 0 | 0 | 1 | 1 | 2 | | | 0 | 0 | 0 | 2 |
| | 0.3 | 0 | 0 | 0 | 0 | 1 | 2 | | | 0 | 0 | 0 | 2 |
| 3 | 10 | | | 0 | 1 | 0 | 2 | 0 | 0 | 0 | 1 | 0 | |
| | 3 | | | 0 | 0 | 0 | 0 | 0 | 0 | 0 | 1 | 0 | |
| | 1 | | | 0 | 0 | 0 | 0 | 0 | 0 | 0 | 0 | 0 | |
| | 0.3 | | | 0 | 0 | 0 | 0 | 0 | 0 | 0 | 0 | 0 | |
| 4 | 10 | 2 | 2 | 2 | 2 | 2 | 2 | | | 2 | 2 | 2 | 2 |
| | 3 | 0 | 2 | 0 | 2 | 2 | 2 | | | 2 | 2 | 2 | 2 |
| | 1 | 0 | 0 | 0 | 0 | 0 | 2 | | | 1 | 2 | 2 | 2 |
| | 0.3 | 0 | 0 | 0 | 0 | 0 | 0 | | | 0 | 0 | 1 | 1 |
| 6 | 10 | | | 2 | 2 | 2 | 2 | 0 | 2 | 2 | 2 | 2 | |
| | 3 | | | 0 | 0 | 0 | 2 | 0 | 0 | 0 | 0 | 0 | |
| | 1 | | | 0 | 0 | 0 | 2 | 0 | 0 | 0 | 0 | 0 | |
| | 0.3 | | | 0 | 0 | 0 | 0 | 0 | 0 | 0 | 0 | 0 | |
| 7 | 10 | | | 0 | 2 | 0 | 2 | 0 | 0 | 0 | 1 | 2 | |
| | 3 | | | 0 | 0 | 0 | 0 | 0 | 0 | 0 | 1 | 0 | |
| | 1 | | | 0 | 0 | 0 | 0 | 0 | 0 | 0 | 0 | 0 | |
| | 0.3 | | | 0 | 0 | 0 | 0 | 0 | 0 | 0 | 0 | 0 | |
| 8 | 10 | 0 | 0 | 0 | 2 | 2 | 2 | | | nd | 2 | | |
| | 3 | 0 | 0 | 0 | 1 | 2 | 2 | | | 0 | 2 | | |
| | 1 | 0 | 0 | 0 | 0 | 1 | 2 | | | 0 | 2 | | |
| | 0.3 | 0 | 0 | 0 | 0 | 1 | 1 | | | 0 | 0 | | |
| 10 | 10 | 2 | 2 | 2 | 2 | 2 | 2 | 2 | | 2 | 2 | 2 | 2 |
| | 3 | 2 | 2 | 0 | 2 | 2 | 2 | 0 | 2 | 2 | 2 | 0 | 2 |
| | 1 | 0 | 2 | 0 | 0 | 2 | 2 | 0 | 0 | 0 | 2 | 0 | 2 |
| | 0.3 | 0 | 0 | 0 | 0 | 1 | 1 | 0 | 0 | 0 | 0 | 0 | 0 |
| 11 | 10 | 0 | 2 | 0 | 2 | 2 | 2 | 0 | 2 | 2 | 2 | | |
| | 3 | 0 | 1 | 0 | 0 | 2 | 2 | 0 | 0 | 0 | 1 | | |
| | 1 | 0 | 0 | 0 | 0 | 0 | 2 | 0 | 0 | 0 | 0 | | |
| | 0.3 | 0 | 0 | 0 | 0 | 0 | 1 | | 0 | 0 | 0 | | |
| 12 | 10 | 2 | 2 | 0 | 2 | 2 | 2 | 0 | 2 | 2 | 2 | | |
| | 3 | 0 | 2 | 0 | 2 | 1 | 2 | 0 | 0 | 0 | 2 | | |
| | 1 | 0 | 0 | 0 | 1 | 0 | 2 | 0 | 0 | 0 | 1 | | |
| | 0.3 | 0 | 0 | 0 | 0 | 0 | 1 | 0 | 0 | 0 | 0 | | |
| 13 | 10 | 2 | 2 | 0 | 2 | 2 | 2 | 0 | 0 | 2 | 2 | | |
| | 3 | 0 | 2 | 0 | 2 | 1 | 2 | 0 | 0 | 0 | 1 | | |
| | 1 | 0 | 0 | 0 | 0 | 0 | 2 | 0 | 0 | 0 | 0 | | |
| | 0.3 | 0 | 0 | 0 | 0 | 0 | 0 | 0 | 0 | 0 | 0 | | |

| No. | conc | H9 | | HBL 100 | | HT 1080 | | PC-3 | | SKMEL 1 | |
|---|---|---|---|---|---|---|---|---|---|---|---|
| | | 24h | 48h | 24h | 48h | 24h | 48h | 24h | 48h | 24h | 48h |
| 2 | 10 | 0 | 0 | 0 | 2 | 0 | 1 | 0 | 2 | 0 | 0 |
| | 3 | 0 | 0 | 0 | 0 | 0 | 0 | 0 | 0 | 0 | 0 |
| | 1 | 0 | 0 | 0 | 0 | 0 | 0 | 0 | 0 | 0 | 0 |
| | 0.3 | 0 | 0 | 0 | 0 | 0 | 0 | 0 | 0 | 0 | 0 |
| 10 | 10 | | | 2 | 2 | 2 | 2 | | | | |
| | 3 | | | 2 | 2 | 2 | 2 | | | | |
| | 1 | | | 2 | 2 | 1 | 2 | | | | |
| | 0.3 | | | 1 | 2 | 0 | 0 | | | | |
| nd: not determined due to self fluorescence of the compound | | | | | | | | | | | |
| 0: no effect | | | | | | | | | | | |
| 1: weak effect | | | | | | | | | | | |
| 2: strong effect | | | | | | | | | | | |

### B) MTS proliferation assay

The assay is performed in 96 well tissue culture plates. The cells (range : 1.5*10³-10⁴) are seeded out in 80 µl complete medium 24 h prior to compound treatment. The test compounds are added in 20 µl complete medium containing max 5% DMSO. The final compound concentrations in the assays are 10 µM, 3 µM,1 µM and 0.3 µM, respectively. The assay plates are incubated for 72 h at culture conditions. The MTS reagent is prepared according to the manufacturer's protocol (Promega G1111 ). 20 µl MTS reagent are added to each well, the assay plates are quickly spun and incubated for another 3 h at culture conditions. Subsequently, the plates are shortly shaked and absorption measured with a microplate-reader at 492 nm. IC₅₀ values are determined by graphical analysis and are indicated in the Table 4 in µM concentration.

**Table 4:**

| MTS proliferation assay | | | | | |
|---|---|---|---|---|---|
| No. | IC 50 | | | | |
| | KB | HeLa | Jurkat | A20 | K562 |
| 4 | | 3 | 3 | | 3 |
| 8 | | 2 | | 2 | 3 |
| 10 | | 2 | 3 | | 3 |
| 11 | | 3 | 3 | | 3 |
| 12 | | 3 | 3 | | 3 |
| 13 | | 3 | 2 | | 3 |
| 1: IC50 < 1 µM | | | | | |
| 2: 1µM<IC50<10µM | | | | | |
| 3: IC50 >10µM | | | | | |

### C) AnnexinV/7-AAD staining

Adherent cells (1 - 2*10⁵) are 24 h prior to compound treatment seeded into 24 well tissue culture plates. Suspension cells are pipetted into P-tubes immediately before treatment. Test compounds are added leading to a final concentrations of 10 µM. After 24 h treatment cells are harvested (in case of adherent cells by trypsinization) and transferred to FACS tubes (BD Biosciences). After centrifugation and removal of the supernatant, 100 µl complete medium containing AnnexinV-GST (10 µg) is added, mixed and incubated at 4°C for 30 minutes. Subsequently, the cells are washed once with medium and incubated with 100 µl anti-GST Alexa 488 (Molecular Probes A-11131) in medium diluted 1:500 for 30 minutes at 4°C. Then, cells are washed once and stained with 1 µg/ml 7-aminoactinomycin D (7-AAD) (Molecular Probes A-1310) in 250 µl medium and analyzed on the FACSCalibur™. AnnexinV is measured in FL1 whereas 7-AAD is measured in FL3. In Table 5 the percentage of positively stained cells after treatment with the respective compounds are indicated. The following scores are used: Score 0 relates to less than 10%, score 1 relates to more than 10% but less than 50% and score 2 relates to more than 50% positivity.

**Table 5:**

| AnnexinV/7-AAD staining (10 µM) | | | | | | |
|---|---|---|---|---|---|---|
| No. | KB | HeLa | Jurkat | A20 | K562 | Ramos |
| 1 | | 1 | 0 | 0 | | 1 |
| 2 | | 1 | 2 | 2 | | 2 |
| 3 | | 1 | 2 | 1 | | 2 |
| 4 | | 1 | 2 | 1 | | 2 |
| 6 | | 2 | 2 | 2 | | 2 |
| 7 | | 1 | 2 | 1 | | 2 |
| 8 | | 1 | 0 | 0 | | 0 |
| 9 | | 1 | 0 | 0 | | 0 |
| 10 | | 2 | 2 | 1 | | 2 |
| 11 | | 1 | 0 | 1 | | 2 |
| 12 | | 2 | 2 | 2 | | 2 |
| 13 | | 1 | 2 | 2 | | 2 |
| 14 | | 1 | 2 | 2 | | 2 |

### D) PI staining for cell cycle distribution

1 - 2*10⁵ cells are seeded into 24 well tissue culture plates and incubated for 24 h prior to compound addition. Compounds are added for 24 h in a final concentration of 3 µM or 10 µM. Adherent cells are harvested by trypsinization. The cell suspensions are fixed by adding 2 parts ice cold ethanol 100% while vortexing. Then the samples are stored for > 2 h at-20°C. Subsequently the cells are washed with PBS once and resuspended in 250 µl PBS containing 50 µg/ml PI (Calbiochem # 537059), then the samples are incubated at 37°C for 30 minutes and subsequently analyzed on a FACSCalibur™ monitoring linear PI fluorescence activity on FL2.. The readout allows the detection of a possible direct or indirect influence of the tested compounds on the cell cycle. The following events can occur: a) Generation of a subG1 peak indicative for DNA fragmentation, b) increase of the cell population arrested in G2M phase. Both events are scored by 1 for weak and 2 for strong occurrence. 0 indicates no occurrence at all. In Table 6 the influences of several tested compounds are demonstrated.

**Table 6:**

| PI staining for cell cycle distribution | | | | |
|---|---|---|---|---|
| No. | HeLa 10 µM | | Jurkat 3 uM | |
| | subG1 | G2M | subG1 | G2M |
| 8 | 1 | 0 | 1 | 0 |
| 10 | 0 | 0 | 0 | 0 |
| 11 | 0 | 0 | 0 | 0 |
| 12 | 0 | 0 | 0 | 0 |
| 13 | 0 | 0 | 0 | 0 |
| 0: no effect | | | | |
| 1: weak effect | | | | |
| 2: strong effect | | | | |

### E) BrdU incorporation (proliferation)

Adherent cells are seeded out at 2 - 4*10⁴ cells/well/ml in 24 well tissue culture plates 24 h prior to treatment. Suspension cells are seeded out at 2*10⁵ cells/ml/well in 24 well plates. Compounds are added leading to final concentrations of 3 µM and 10 µM, respectively. Subsequently, BrdU (Molecular Probes #B-23151) at 10 µM final concentration is added and the plates are incubated for 48 h. After the incubation cells are processed according to standard procedures. The detection of the incorporated BrdU is done with the anti-bromodeoxyuridine Mab PRB-1, Alexa Fluor 660 conjugate (Molecular Probes #A-21306). The analysis is performed on a FACSCalibur™ by monitoring the fluorescence activity on FL3. The readout reflects DNA synthesis which is a hallmark for proliferation. The scores are defined the following: 0 for no activity, 1 for a moderate inhibition and 2 for strong inhibition of proliferation. Table 7 indicates inhibitory activities of several tested compounds on proliferation.

**Table 7:**

| BrdU incorporation | | | | |
|---|---|---|---|---|
| No. | HeLa | | Jurkat | |
| | 3uM | 10uM | 3uM | 10uM |
| 10 | 0 | 0 | 2 | 1 |
| 11 | 1 | | 0 | 2 |
| 12 | 1 | 1 | 2 | 2 |
| 13 | 1 | | 2 | 2 |
| 0: no effect | | | | |
| 1: weak effect | | | | |
| 2: strong effect | | | | |

### F) Caspase dependencies

Caspase dependencies are being evaluated by combining the compound treatment with the pan-caspase inhibitor zVAD or its control peptide zFA (ICN Pharmaceuticals # FK009 and FK029, respectively). Both peptides are being used at 20 µM concentration. In case of caspase dependencies a clear inhibition of the specific readout in all apoptosis tests should be detected. By comparing the readout of zVAD and zFA treated samples with the compound control it is possible to detect caspase resp. cystein proteinase dependencies. In case of inhibition by zVAD but not by zFA a clear caspase dependency is obvious. An inhibition by zVAD as well as by zFA points towards the involvement of cystein proteinases in the apoptotic cascade. Table 8 demonstrates the protease dependency of the nuclear fragmentation visualized by Hoechst 33342 staining.

**Table 8:**

| Caspase dependencies | | | | | | | | | | | | |
|---|---|---|---|---|---|---|---|---|---|---|---|---|
| No. No. | conc cone | treatment treatment | KB | | HeLa | | Jurkat | | A20 | | K562 | |
| | | | 24h | 48h | 24h | 48h | 24h | 48h | 24h | 48h | 24h | 48h |
| 2 | 10 | | 2 | 2 | 2 | 2 | | | | | 2 | 2 |
| | | zFA | 1 | 2 | 2 | 2 | | | | | 2 | 2 |
| | | zVAD | 1 | 2 | 1 | 2 | | | | | 2 | 2 |
| | 3 | | | | | | 2 | 2 | | | | |
| | | zFA | | | | | 2 | 2 | | | | |
| | | zVAD | | | | | 2 | 2 | | | | |
| nd: not determined due to self fluorescence of the compound | | | | | | | | | | | | |
| 0: no effect | | | | | | | | | | | | |
| 1: weak effect | | | | | | | | | | | | |
| 2: strong effect | | | | | | | | | | | | |

### Example 13

### Soft Capsules

5000 soft gelatin capsules, each comprising as active ingredient 0.05 g of one of the compounds of formula I mentioned in the preceding Examples, are prepared as follows: 250 g pulverized active ingredient is suspended in 2 liter Lauroglykol® (propylene glycol laurate, Gattefossé S.A., Saint Priest, France) and ground in a wet pulverizer to produce a particle size of about 1 to 3 µm. 0.419 g portions of the mixture are then introduced into soft gelatin capsules using a capsule-filling machine.

## Claims

1. A compound of formula I, 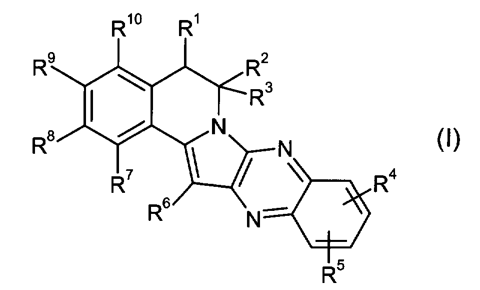 wherein
R¹ represents hydrogen, lower alkyl, lower alkoxy-lower alkyl, halo-lower alkyl or optionally substituted phenyl;
R² represents hydrogen, lower alkyl, lower alkoxy-lower alkyl, halo-lower alkyl, optionally substituted phenyl, or R¹ and R² together with the bond connecting them represent a carbocyclic ring;
R³ represents hydrogen or lower alkyl, or R² and R³ together with the bond connecting them represent a carbocyclic ring;
R⁴ represents hydrogen, lower alkyl, halo-lower alkyl, lower alkoxy, lower alkoxy-lower alkoxy or optionally substituted phenyl;
R⁵ represents hydrogen, lower alkyl, halo-lower alkyl, lower alkoxy, or R⁴ and R⁵ in ortho position to each other represent methylenedioxy or together with the bond connecting them represent an annulated aromatic or aliphatic ring;
**R**^{**6**} represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl;
R⁷, R⁸, R⁹ and R¹⁰, independently of each other, represent hydrogen, lower alkyl, halogen, haloalkyl, lower alkoxy, lower alkylthio, lower alkoxy-lower alkoxy, aminoalkoxy wherein the nitrogen atom is unsubstituted or substituted by lower alkyl, hydroxy-lower alkyl or lower alkoxy-lower alkyl or wherein the nitrogen atom is part of heterocyclyl, lower acyloxy, lower alkoxy-lower acyloxy, or di-lower alkylamino-lower acyloxy; or also R⁷ and R⁸, or R⁸ and R⁹, or R⁹ and R¹⁰ together represent methylenedioxy or an annulated aromatic or aliphatic ring; with the proviso that at least one of the substituents R⁷, R⁸, R⁹ and R¹⁰ represents hydrogen; and salts thereof;
with the exception of compounds of formula I wherein
R¹ is hydrogen, R² and R³ are both methyl, R⁴, R⁵, R⁶ and R⁷ are hydrogen, R⁸ and R⁹ are both hydrogen or both methoxy, and R¹⁰ is hydrogen;
R¹ is hydrogen, R² and R³ together represent tetramethylene, and R⁴, R⁵, R⁶, R⁷, R⁸, R⁹, and
R¹⁰ are hydrogen; and wherein
R¹ and R² together represent tetramethylene, R³ is methyl, and R⁴, R⁵, R⁶, R⁷, R⁸, R⁹, and
R¹⁰ are hydrogen.

2. A compound of formula I according to claim 1 wherein
R¹ represents hydrogen, lower alkyl, lower alkoxy-lower alkyl or optionally substituted phenyl;
R² represents hydrogen, lower alkyl, lower alkoxy-lower alkyl, optionally substituted phenyl, or R¹ and R² together with the bond connecting them represent a carbocyclic ring;
R³ represents hydrogen or lower alkyl, or R² and R³ together with the bond connecting them represent a carbocyclic ring;
R⁴ and R⁵ represent hydrogen;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl;
R⁷, R⁸, R⁹ and R¹⁰, independently of each other, represent hydrogen, lower alkyl, halogen, haloalkyl, lower alkoxy, lower alkylthio, lower alkoxy-lower alkoxy, aminoalkoxy wherein the nitrogen atom is unsubstituted or substituted by lower alkyl, hydroxy-lower alkyl or lower alkoxy-lower alkyl or wherein the nitrogen atom is part of heterocyclyl, lower acyloxy, lower alkoxy-lower acyloxy, or di-lower alkylamino-lower acyloxy; or also R⁷ and R⁸, or R⁸ and R⁹, or R⁹ and R¹⁰ together represent methylenedioxy or an annulated aromatic or aliphatic ring; with the proviso that at least one of the substituents R⁷, R⁸, R⁹ and R¹⁰ represents hydrogen; and salts thereof.

3. A compound of formula I according to claim 1 wherein
R¹ represents hydrogen;
R² represents hydrogen, lower alkyl or optionally substituted phenyl;
R³ represents hydrogen or lower alkyl, or R² and R³ together with the bond connecting them represent a carbocyclic ring;
R⁴ and R⁵ represent hydrogen;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl;
R⁷, R⁸, R⁹ and R¹⁰, independently of each other, represent hydrogen, lower alkyl, halogen, haloalkyl, lower alkoxy, lower alkylthio, lower alkoxy-lower alkoxy, aminoalkoxy wherein the nitrogen atom is unsubstituted or substituted by lower alkyl, hydroxy-lower alkyl or lower alkoxy-lower alkyl or wherein the nitrogen atom is part of heterocyclyl, lower acyloxy, lower alkoxy-lower acyloxy, or di-lower alkylamino-lower acyloxy; or also R⁷ and R⁸, or R⁸ and R⁹, or R⁹ and R¹⁰ together represent methylenedioxy or an annulated aromatic or aliphatic ring; with the proviso that at least one of the substituents R⁷, R⁸, R⁹ and R¹⁰ represents hydrogen; and salts thereof.

4. A compound of formula I according to claim 1 wherein
R¹ represents hydrogen;
R² represents hydrogen or lower alkyl;
R³ represents hydrogen or lower alkyl, or R² and R³ together with the bond connecting them represent a carbocyclic ring;
R⁴ and R⁵ represent hydrogen;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl;
R⁷, R⁸, R⁹ and R¹⁰, independently of each other, represent hydrogen, lower alkyl or lower alkoxy, or R⁸ and R⁹ together represent methylenedioxy, with the proviso that at least one of the substituents R⁷, R⁸, R⁹ and R¹⁰ represents hydrogen;
and salts thereof.

5. A compound of formula I according to claim 1 wherein
R¹ represents hydrogen;
R² represents hydrogen or lower alkyl;
R³ represents hydrogen or lower alkyl, or R² and R³ together with the bond connecting them represent a carbocyclic ring;
R⁴ and R⁵ represent hydrogen;
R⁶ represents hydrogen, alkyl, optionally substituted phenyl, halogen, hydroxyalkyl, alkoxyalkyl, or trisubstituted silyl wherein the substituents are lower alkyl or phenyl;
R⁷, R⁸, R⁹ and R¹⁰, independently of each other, represent hydrogen, lower alkyl or lower alkoxy, or R⁸ and R⁹ together represent methylenedioxy, with the proviso that at least one of the substituents R⁷, R⁸, R⁹ and R¹⁰ represents hydrogen;
and pharmaceutically acceptable salts thereof.

6. A compound of formula I, 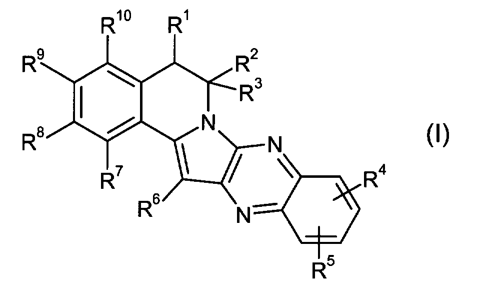 wherein
R¹ represents hydrogen, lower alkyl, lower alkoxy-lower alkyl or halo-lower alkyl;
R² represents hydrogen, lower alkyl, lower alkoxy-lower alkyl, halo-lower alkyl, or R¹ and R² together with the bond connecting them represent a carbocyclic ring;
R³ represents hydrogen or lower alkyl, or R² and R³ together with the bond connecting them represent a carbocyclic ring;
R⁴ represents hydrogen, lower alkyl, halo-lower alkyl, lower alkoxy, lower alkoxy-lower alkoxy or optionally substituted phenyl;
R⁵ represents hydrogen, lower alkyl, halo-lower alkyl, lower alkoxy, or R⁴ and R⁵ in ortho position to each other represent methylenedioxy or together with the bond connecting them represent an annulated aromatic or aliphatic ring;
R⁶ represents hydrogen, alkyl, cycloalkyl, cycloalkylalkyl, optionally substituted arylalkyl, optionally substituted alkenyl, optionally substituted alkinyl, optionally substituted phenyl, nitro, cyano, halogen, haloalkyl, hydroxyalkyl, alkoxyalkyl, aryloxyalkyl, arylalkoxyalkyl; aminoalkyl wherein the nitrogen atom is unsubstituted or substituted by one or two groups selected from alkyl, cycloalkyl, cycloalkyl-lower alkyl, alkenyl, alkinyl, hydroxy-lower alkyl, lower alkoxy-lower alkyl, optionally substituted phenyl, or wherein the nitrogen is part of heterocyclyl; formyl, alkylcarbonyl, cycloalkylcarbonyl, cycloalkylalkylcarbonyl, alkoxyalkylcarbonyl, haloalkylcarbonyl, optionally substituted aminoalkylcarbonyl, optionally substituted arylcarbonyl, or corresponding acetals wherein the carbonyl group is present as dimethoxy acetal, ethylenedioxyacetal or propylenedioxyacetal; hydroxycarbonyl, lower alkoxycarbonyl, carbamoyl; substituted carbamoyl wherein one substituent is lower alkyl, lower alkenyl, lower alkoxy-lower alkyl, lower dialkylamino-lower alkyl or optionally substituted phenyl and the other substituent is hydrogen or lower alkyl; hydroxysulfonyl; substituted thio, sulfinyl or sulfonyl wherein the substituent is lower alkyl, haloalkyl, cycloalkyl or optionally substituted phenyl; or trisubstituted silyl wherein the substituents are lower alkyl or phenyl;
R⁷, R⁸, R⁹ and R¹⁰, independently of each other, represent hydrogen, lower alkyl, halogen, haloalkyl, lower alkoxy, lower alkylthio, lower alkoxy-lower alkoxy, aminoalkoxy wherein the nitrogen atom is unsubstituted or substituted by lower alkyl, hydroxy-lower alkyl or lower alkoxy-lower alkyl or wherein the nitrogen atom is part of heterocyclyl, lower acyloxy, lower alkoxy-lower acyloxy, or di-lower alkylamino-lower acyloxy; or also R⁷ and R⁸, or R⁸ and R⁹, or R⁹ and R¹⁰ together represent methylenedioxy or an annulated aromatic or aliphatic ring; with the proviso that at least one of the substituents R⁷, R⁸, R⁹ and R¹⁰ represents hydrogen; and pharmaceutically acceptable salts thereof; for use as a medicament.

7. A method for the preparation of a compound of formula I according to claim 1 or 6, wherein R¹ to R¹⁰ are defined as in claim 1, **characterized in that** a compound of formula II 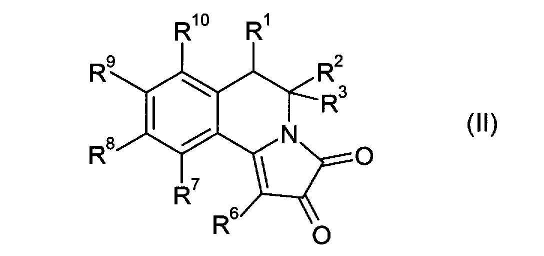 wherein R¹ to R³ and R⁶ to R¹⁰ are as defined for a compound of formula I, is reacted with an optionally substituted o-phenylenediamine of formula III 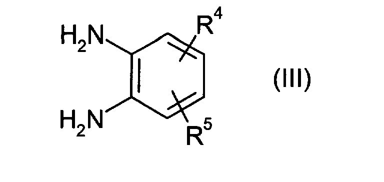 wherein R⁴ and R⁵ are as defined for a compound of formula I, optionally in the presence of a dehydrating agent and an inert base, or an inert acid, and/or a suitable catalyst, and optionally in the presence of an inert solvent; where the above starting compounds of formula II and III may also be present with functional groups in protected form if necessary and/or in the form of salts, provided a salt-forming group is present and the reaction in salt form is possible; any protecting groups in a protected derivative of a compound of the formula I are removed; and, if so desired, an obtainable compound of formula I is converted into another compound of formula I, a free compound of formula I is converted into a salt, an obtainable salt of a compound of formula I is converted into the free compound or another salt, and/or a mixture of isomeric compounds of formula I is separated into the individual isomers.

8. A pharmaceutical composition comprising a compound of formula I as defined in claim 1 or 6 or a pharmaceutically acceptable salt of such a compound and a pharmaceutically acceptable carrier.

9. Use of a compound of formula I as defined in claim 1 or 6 or a pharmaceutically acceptable salt of such a compound for the preparation of a pharmaceutical composition for the treatment of a neoplastic disease, autoimmune disease, transplantation related pathology and/or degenerative disease.
